# EUROPEAN PATENT APPLICATION

(11) **EP 4 631 557 A1**
(43) Date of publication of application: **15.10.2025**
(21) Application number: 24169027.0
(22) Date of filing: 08.04.2024
(51) Int. Cl.: A61M 16/06

(54) **MINIMAL CONTACT RESPIRATORY NASAL MASK WITH IMPROVED LATERAL ARM STRUCTURE**

(71) Applicant: Air Liquide Medical Systems, 92182 Antony Cedex (FR)
(72) Inventor: ALBERICI, Luca, 25073 Bovezzo (IT); ROMAIOLI, Roberto, 25073 Bovezzo (IT); RUOCCO, Alessandra, 25073 Bovezzo (IT); LEBRET, Marius, 92182 Antony (FR)
(74) Representative: Air Liquide

(57) **Abstract**

A respiratory nasal mask (1) comprising a mask body (10) comprising a front opening (11) with a peripheral rim (18), and two elongated arms (14), each elongated arm comprising a longitudinal axis (XX), with the two elongated arms attached to opposite lateral sides (11a, 11b) of the mask body (10), and a hollow flexible cushion (20) comprising a supple rear part (21) comprising a rear breathing opening for allowing, in use, gas exchanges with nostrils of a user (P), the hollow flexible cushion (20) being detachably attached to the mask body (10). The peripheral rim (18) of the front opening (11) of the mask body (10) is comprised in a first plane (P1), and the two longitudinal axis (XX) of the elongated arms (14) are comprised in a second plane (P2), the first plane (P1) and the second plane (P2) being inclined with respect to each other with an inclination angle (A) of less than 90°.

## Description

The invention concerns a respiratory nasal mask comprising a mask body and a flexible nasal cushion detachably coupled or fixed together, that is useable in the treatment of respiratory conditions or diseases affecting adults, children, toddlers, infants or newborns, such as obstructive sleep apnea (OSA).

Respiratory masks are commonly used for delivering a flow of breathable gas, such as air under pressure, for, or to assist in, patient respiration.

A mask assembly typically comprises a rigid or semi-rigid hollow shell or body, usually made of polymer, and further comprising a soft face-contacting cushion, commonly called a flexible cushion, that comes into contact with the patient's face and conforms to the various facial contours of the patient face for receiving the patient's nose and/or mouth so that the patient can inhale the gas comprised into a breathing chamber that is defined by the hollow shell or body and/or the flexible cushion. The flexible cushion is usually made of soft, resilient elastomeric material, such as soft silicone or similar material.

A gas supply line delivers a respiratory gas, such as air under pressure, to the mask, namely the breathing chamber. An example of such a mask is given by EP-A-462701.

Further, a headgear, comprising flexible straps or similar structures, is usually attached to the mask for correctly positioning, holding and/or securing the mask on the head of a patient. The headgear can be adjusted to pull the mask against the face with sufficient force to achieve a gas tight seal between the mask and the wearer's face as taught by EP-A-462701, EP-A-874667, EP-A-1972357 or WO-A-00/57942.

Other types of masks comprising a front shroud adapted to attach the headgear are disclosed by EP-A-2708258 or US-A-2007/0044804.

Masks receiving only the patient's nose are called a nasal mask, whereas those receiving the patient's nose and mouth are called facial or oro-nasal masks. Further, masks contacting only the outer regions located around the nostrils of the user are called minimal contact masks (MCM). An example is disclosed by EP4279108.

Generally speaking, masks can be used for treating various respiratory conditions or diseases affecting adults or children, including some toddlers, infants or newborns, such as obstructive sleep apnea (OSA) or other pathologies. In particular, the effective treatment of OSA requires that the patient uses the nasal mask overnight, namely when he/she sleeps, which leads to various problems.

Indeed, despite the fact that a lot of different architectures or masks have been proposed in the prior art, especially for treating OSA or the like, problems or drawbacks still exist with existing nasal masks. Thus, one important problem is related to the discomfort of use for the patient.

Various causes of discomfort exist, such as gas leaks due to a wrong assembling of the mask components, a poor positioning of the mask of the patient's face, loose settings of the headgear, poor tightness of the cushion, especially overnight...

A goal of the present invention is to provide an improved minimal contact mask (MCM) that ensures efficient positioning and securing of the mask on the patient's face, and an efficient gas tightness, i.e. seal, preventing the escape of gas, i.e. gas leaks, when donned by a user, including overnight.

Preferably, the improved MCM according to the present invention should also be compact, simple to assemble/disassemble, light and comfortable to wear, and comfortable in use for the patients.

Preferably, the improved MCM according to the present invention should be useable for treating OSA or other pathologies.

A solution according to the present invention concerns a respiratory nasal mask, namely a so-called "minimal contact mask" (MCM), comprising:
- a mask body comprising with a front opening, i.e. orifice or the like, with a peripheral rim, i.e. a flange, an edge or the like, and two elongated arms each comprising a longitudinal axis (XX) attached to opposite lateral sides, i.e. left and right sides, of the mask body, and
- a hollow flexible cushion comprising a supple rear part comprising a rear breathing opening for allowing, in use, gas exchanges with nostrils of a user, i.e. a patient, said supple rear part comprising peripheral edge regions surrounding the rear breathing opening that are configured to, in use, come into contact and provide a gas sealing with areas located immediately around the nostril edges of the user.

Further, the hollow flexible cushion is detachably attached to the mask body.

Furthermore, according to the invention, the peripheral rim of the front opening of the mask body is comprised in a first plane (P1), and the two longitudinal axis (XX) of the elongated arms are comprised in a second plane (P2), wherein the first plane (P1) and the second plane (P2) are inclined with respect to each other with an inclination angle (A) of less than 90°, i.e. they are not perpendicular.

The respiratory nasal mask according to the present invention can further comprise one or more of the following features.
- the inclination angle (A) is of at least 60°.
- the inclination angle (A) is of at least 65°.
- the inclination angle (A) is of less than 88°.
- the inclination angle (A) is of less than 86°.
- the inclination angle (A) is comprised between 70° and 85°.
- the two lateral arms project, one on each lateral side of the mask body (10), toward the cushion so that the cushion is sandwiched between said two lateral arms.
- the two lateral arms are made one-piece with the mask body.
- the mask body is made of polymer material.
- the supple rear part of the flexible cushion comprises an upper lip area (ULA) located immediately under the nostrils of the patient (P), two curved alae (CA) on both sides of the nostrils of the patient (P) and a tip region (TR) of the nose of the patient (P), when the mask (1) is donned by the patient (P).
- the cushion is made of silicone.
- a tubular hollow connector is secured to the front opening of the mask body, preferably rotatably secured to the front opening.
- the peripheral rim of the front opening has a cylindrical section.
- the two lateral arms comprise headgear-connecting structures, preferably slots or hooks.
- a headgear is attached to the headgear-connecting structures.
- the headgear comprises elastic or flexible straps.
- the two lateral arms and the mask body are made of a rigid or semi-rigid polymer material.
- the rigid or semi-rigid polymer material, i.e. a plastic material, is polycarbonate (PC), polypropylene (PP), Nylon^{®}, or the like, for instance PC.
- the two lateral arms have an elongated shape, such as a band or ribbon shape. Other shapes are of course also possible.
- the lateral arms have each a length of about 3 to 10 cm, preferably of about 3 to 6 cm.
- each lateral arm comprise a free end comprising the headgear-connecting structures for attaching a headgear thereto.
- the headgear connecting structures comprise one or several slots, holes, hooks or the like.
- the mask body comprises a front wall traversed by the front opening, preferably a curved front wall.
- the mask body comprises two opposite surfaces, namely a front surface (i.e. toward the outside) and a rear surface facing the cushion (i.e. toward the inside).
- the front wall has a length (L) of between 40 and 70 mm and a width (W) of between 30 and 50 mm.
- the front wall has a thickness of between 2 and 15 mm.
- the front wall is flat.
- the front wall is slightly foldable, i.e. can be slightly bent, especially when tension forces are applied by the headgear connected to the two arms.
- the front wall comprises two opposite tips or sides, i.e. right and left sides, the two lateral arms being attached to said two opposite tips or sides.
- the front opening is arranged at the center of the front wall, namely constitutes a central opening.
- the front opening has a circular shape, i.e. a circular section.
- the front opening has a diameter of between 15 and 25 mm, preferably of between 17 and 22 mm.
- the front wall of the mask body further comprises several venting orifices, i.e. holes, slots or similar, traversing the front wall and arranged around the front opening.
- the venting orifices are in fluid communication with the inner breathing chamber of the flexible nasal cushion.
- the venting orifices are arranged in groups or arrays comprising each several venting orifices.
- groups or arrays of venting orifices are arranged so as to sandwich the front opening of the mask body, i.e. arranged to the left and to right of the front opening.
- the venting orifices traverse the front wall and open on the rear surface of the mask body between the collar structure and the tubular rear section of the mask body.
- the venting orifices are configured for venting to the atmosphere at least a part of the CO₂-containing gaseous flow expired by the user during the expiratory phases.
- the venting orifices have a cylindrical or tronconical shape, preferably their section is greater on the rear surface of the mask body than on the front surface of said mask body.
- the flexible cushion comprises an inner breathing chamber, i.e. an inner room or compartment for the gases, i.e. gas delivered to the user, such as a patient, during inspiratory phases and/or CO₂-enriched gases exhaled by the user during expiratory phases.
- the cushion is sized and designed for not covering the regions of the nose of the user situated above his/her nose tip or tip region (TR), when the mask is worn by said user, especially the dorsum, i.e. cartilaginous and/or bony dorsum.
- the cushion is made of a flexible material, i.e. a supple and soft or semi-soft material, such as silicone or the like.
- the rear opening is in fluid communication with the inner breathing chamber.
- the front opening of the mask body is in fluid communication with the inner breathing chamber of the flexible cushion
- the rear opening of the cushion has (almost or quasi) rectangular or trapezoidal shape.
- the peripheral edge regions surrounding the rear opening of the flexible cushion comprise a peripheral thin wall, such as a membrane or the like, constituting a sealing, preferably a supple sealing skirt, around said rear opening so as to ensure an efficient gas tightness while in contact with the user's face, when the latter wears the mask, preferably all along the outer periphery of said rear opening.
- the peripheral thin wall of the peripheral edge regions of the cushion forming the sealing comes into contact and provide a gas sealing with the areas located immediately around the nostril edges of the use, namely the upper lip area (ULA), the two curved alae (CA), and the tip region (TR) of the nose.
- the rear surface of the flexible cushion is curved, in particular for matching, in use, the two curved alae (CA) of the nose of the user.
- the peripheral edge regions surrounding the rear opening of the flexible nasal cushion form a curved (quasi) contacting surface, preferably a triangular or trapezoidal curved contacting surface.
- the curved (quasi) contacting surface surrounding the rear opening comes into contact, in use, i.e. when the user wears the mask, with the areas (i.e. user's facial areas) located immediately around the nostril edges of the user thereby providing an efficient gas sealing.
- the peripheral edge regions of the rear opening of the flexible cushion comprises a base border, two lateral panels or wings, and a top border.
- the width of the top border is larger/greater than the width of the base border, for instance the width of the top border is between 17 and 25 mm, whereas the width of the base border is between 10 and 20 mm.
- the peripheral thin wall forming the sealing skirt around the rear breathing opening is molded in one piece with the rest of the cushion.
- a bridge element or support-element is arranged in the rear opening.
- the bridge element has an arch-shape or the like that projects into the cushion.
- the bridge element has a "U" or "V"-shape or similar.
- the bridge element forms a bridge between the upper and lower edges of the rear opening.
- when the mask is worn, the bridge element is in contact with the outer region of the nose of the patient separating the nostrils so as to constitute a support or the like prohibiting a collapse, sagging or similar of the thin regions of the cushion, namely the peripheral edge regions of the rear opening of the flexible cushion, in particular the base and top borders.
- the peripheral edge regions of the rear opening of the flexible cushion are thinner than the tubular front part regions of the flexible cushion.
- the thickness of the wall of the flexible cushion in the peripheral edge regions of the rear opening is less than 1mm, preferably of between 0.30 and 0.6 mm.
- the flexibility of the cushion is greater in the peripheral edge regions of the rear opening than in the tubular front part regions of said flexible cushion.
- the mask body and the cushion comprise connecting structures, i.e. fastening means, for attaching the cushion directly to the mask body, in a detachable manner so that the user can disassemble them for cleaning operations for instance.
- the fastening means comprise an annular shoulder or flange arranged in the mask body cooperating with an annular groove arranged in the flexible cushion.
- the annular shoulder or flange of the mask body is lodged and retained into the annular groove of the flexible cushion, when the hollow flexible cushion is attached to the mask body.
- a flexible hose is connected to the front opening of the mask body by means of a hollow connector.
- the hollow connector is a straight connector, i.e. tubular shape, preferably having a cylindrical shape.
- the hollow connector comprises an inner passage for the gas, i.e. a lumen.
- the hollow connector is inserted into, i.e. plugged, and held in position in the mask body, through the front opening of the mask body.
- the hollow connector is detachably fixed to the mask body.
- the hollow connector is rotatable with respect to the mask body.
- the hollow connector is configured for connecting a flexible hose thereto, i.e. a gas line or the like. In other words, the tubular connector is adapted for receiving a flexible conduct or hose that feeds a respiratory gas under pressure to the respiratory mask. The gas is fed into the flexible gas conduct or hose by a gas source, such as a medical ventilator, a medical gas-delivery device or the like.
- the hollow connector is in fluid communication with the breathing chamber of the cushion thereby allowing gas to circulate from the hollow connector to the cushion or in the opposite way.
- the hollow connector is inserted and firmly hold into the front opening of the mask body, i.e. secured therein, for instance by a snap-fit connection, a press-fit connection or others
- the hollow connector has a cylindrical or tronconical shape.
- according to another embodiment, the connector is a hollow curved or elbow connector, i.e. having a "L-shape" or the like.
- it is made of polymer material, e.g. plastic.
- the headgear comprises elastic and/or flexible straps that are used for positioning, maintaining and/or securing the mask in a desired position on the facial regions of the patient.
- the straps are made of polymer material or fabric material, or both.
- the straps comprise or are elongated elastic and/or flexible bands, ribbons or the like.
- the headgear constitutes a tridimensional structure that is installed on the head of the user.
- the straps comprise Velcro^{®}-like portions for forming loops or the like and allowing attaching the headgear to the headgear connecting structures of the mask body.

The invention also concerns a respiratory assembly or installation for providing gas to a patient (P) comprising a gas delivery device, such as a medical ventilator, i.e. a respiratory assistance device or the like, a nasal mask according to the present invention, and a headgear attached to the mask, wherein the gas delivery device is connected to the nasal mask by means of the flexible hose.

Such a respiratory assembly comprising a nasal mask according to the present invention is usable in the treatment of respiratory conditions or diseases affecting adults, children, toddlers, infants or newborns, such as obstructive sleep apnea (OSA).

A non-limitative embodiment of a respiratory nasal mask according to the present invention is shown in the enclosed Figures, among which:
- Figure 1 represents a front view of a patient equipped with a nasal mask,
- Figure 2 shows an embodiment of a nasal mask according to the present invention.
- Figure 3 is lateral view of the mask of Fig. 2.
- Figure 4 shows a nasal mask according to the present invention donned by a patient.
- Figure 5 illustrates the planes and the angle in-between of a nasal mask according to the present invention.

The present invention proposes a respiratory nasal mask 1, also called a minimal contact mask (MCM), as shown in Figures that illustrate an embodiment of a nasal mask 1 according to the present invention.

A nasal mask 1 according to the present invention comprises two main parts assembled or coupled together, namely a mask body 10 and a flexible cushion 20 attached together in a detachable manner so that they can be disassembled for instance for cleaning operations or for replacing a worn out cushion 20, and easily reassembled afterwards.

This mask 1 has a simple and light structure, and further facilitates the assembling/disassembling of the different components of the mask 1 by the users, namely the patients, especially during daily cleaning operations. Such a mask 1 further exhibits a good comfort as it is lighter and has a low risk of gas leaks.

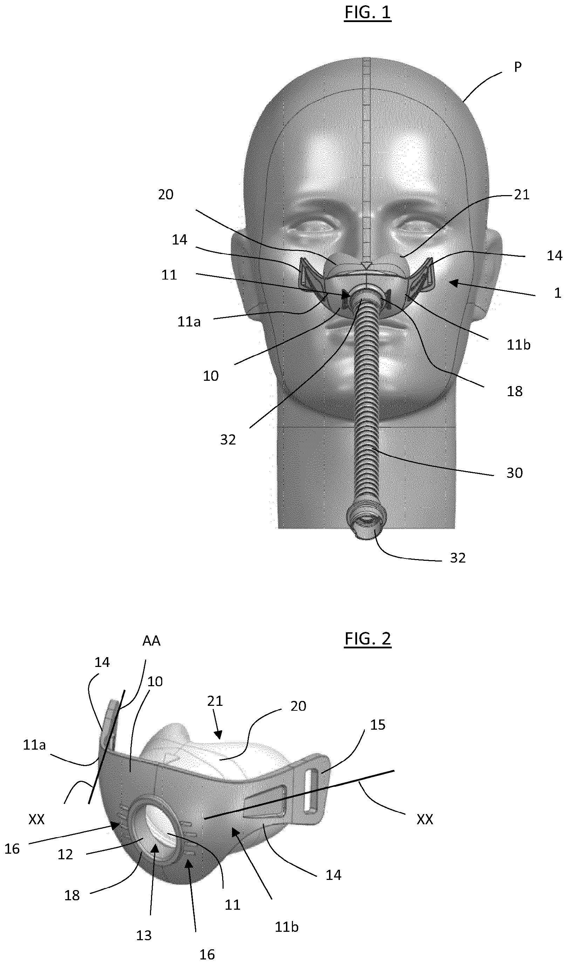

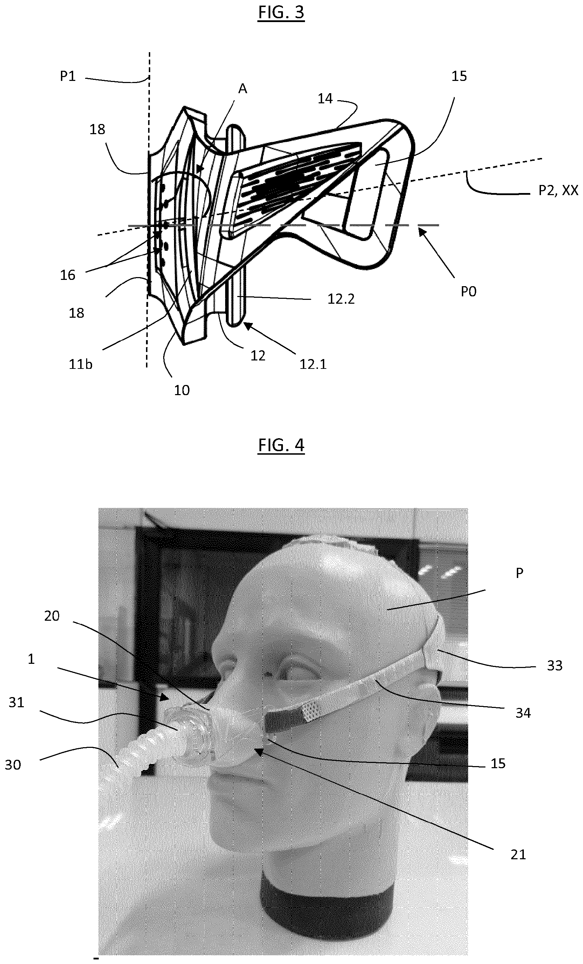

As shown in Fig. 1 & 4, a respiratory gas, such as air, is provided by a flexible hose 30 fluidly and mechanically connected to the mask body 10 by means of a tubular connector 31 arranged at the proximal end of said flexible hose 30. The distal end of the flexible hose 30 comprises an additional tubular connector 32 for fluidly and mechanically connecting the flexible hose 30 to a medical ventilator (not shown) or a similar apparatus that provides the respiratory gas to the flexible hose 30.

A nasal mask 1 of the present invention comprises a mask body 10 comprising two lateral arms 14 attached to opposite lateral sides 11a, 11b of the mask body 10, namely to the left side 11a and to the right side 11b as shown in Fig. 1. The two lateral arms 14 further project toward the rear side of the mask 1, i.e. so as to "sandwich" the cushion 20 as shown in Fig. 1 & 4.

A front opening 11 traverses the mask body 10, preferably the front opening 11 is arranged at the center of the mask body 10. The front opening 11 comprises a rim 18 or outer border. Preferably, the rim 18 has a circular section or the like.

The two lateral arms 14 that project on the left and the right sides of the masks 1 may also constitute right and left cheek supports, when the mask is worn by a patient, as shown in Fig. 4.

According to the invention, the two lateral arms 14 have elongated shapes, such as band or ribbon shapes. Each arm 14 comprises a longitudinal axis (XX) as illustrated in Fig. 2 & 3.

According to some embodiments, the two lateral arms 14 can be slightly incurved to better match, if desired, some of the contours of the cushion 20.

Each lateral arm 14 can have for instance a length of about 3 to 10 cm, preferably of about 3 to 6 cm.

Further, the lateral arms 14 comprise a free end equipped with headgear connecting structures 15, such one or several slots, holes, hooks or the like. These headgear-connecting structures 15 are designed for attaching a headgear 33 thereto as shown in Fig. 4, typically the flexible/elastic straps 34 of a headgear 33. A headgear 33 is commonly used for maintaining and securing the mask 1 in a desired position on the head of the patient P, as explained below. Preferably, the straps 34 of the headgear 33 are made of polymer or fabric materials that can be slightly elastic or the like.

Further, the front opening 11 of the mask body 10 constitutes the entry of a conduct 12 comprising an inner passage 13, i.e. a lumen or the like, in fluid communication with the inner chamber of the cushion 20. The conduct 12 projects away or protrudes from the rear surface or side of the mask body 10, i.e. toward the cushion 20.

Fastening means arranged on the rear surface or side of the mask body 10 and on the cushion 20 are used for firmly securing the cushion 20 to the mask body 10. As taught by EP427910, said fastening means can comprise at least an end portion 12.1 of the conduit 12 as shown in Fig. 3, which comprises an annular shoulder or flange 12.2, and further an annular groove (not shown) arranged in a front opening (not shown) of the cushion 20. The annular shoulder 12.2 of the mask body 10 cooperates with the annular groove of the cushion 20, preferably the annular shoulder 12.2 is lodged in the annular groove, for securing the cushion 20 to the mask body 10. In other words, the end portion 12.1 of the conduct 12 is plugged into the front opening of the cushion 20 and firmly maintained therein. Preferably, a collar structure coaxially arranged around the conduit 12 can be provided for improving the attachment of the cushion 20 to the mask body 10.

Generally speaking, the flexible cushion 20 is detachably fixed to the mask body 10 for facilitating a replacement or a cleaning of the cushion and/or of the mask body 10.

Further, the hollow flexible cushion 20 comprises an inner chamber for the gas and a supple rear part 21 comprising a rear breathing opening for allowing, in use, gas exchanges with the two nostrils of a user P as below detailed. The inner chamber is located between the front opening and the rear opening of the cushion 20 thereby allowing a fluid communication from the front opening to the rear opening, via the inner chamber, typically during inspiration phases, and vice versa, typically during expiration phases.

The flexible cushion 20 constitutes a tridimensional hollow flexible structure defining the inner breathing chamber.

As the mask is a MCM, the supple rear part 21 of the flexible cushion 20 comprises peripheral edge regions surrounding the rear breathing opening that are configured to, in use, come into contact and provide a gas sealing with areas located immediately around the nostril edges of the user P comprising an upper lip area (ULA) located immediately under the nostrils, two curved alae (CA) on both sides of the nostrils and a tip region (TR) of the nose, thereby providing a minimal contact surface or the like with facial areas of the user. In other words, the regions located immediately around the rear breathing opening of the cushion 20 constitute contact zones contacting said specific nasal areas of the user.

The cushion 20 of the nasal mask 1 of the present invention has a compact size and does not extend over the top of the nose, namely does not cover the dorsum regions, i.e. cartilaginous and/or bony dorsum, when the mask 1 is worn by a user P as shown in Fig. 1 & 4, which reduces and/or minimizes the contact with the skin of the user as it rests only on the areas located immediately around the entrances of the nostrils of said user. In other words, the compact cushion 20 of the invention can be sized and/or designed to take into account only the width of the bottom of the nose of the user.

In order to provide efficient gas tightness (i.e. seal) and/or good comfort for the patient, the cushion wall in the peripheral edge regions surrounding the rear opening of the supple rear part 21 of the cushion 20 is preferably designed or conceived as a thin membrane, i.e. a soft flexible membrane, that comes into contact with the patient's facial areas or regions, namely the upper lip area (ULA), the two curved alae (CA) and tip region (TR) as above stated, so as to better match his/her facial morphology. Said flexible thin membrane forms a soft skirt or the like all along the edges of the rear opening of the cushion 20. A unique membrane is preferably used; however, in alternative embodiments, several membranes may be superimposed.

Preferably, the rear outer surface of the cushion 20 that comprises the peripheral edge regions surrounding the rear breathing opening, has a curved shape for better matching some nasal regions, in particular the two curved alae (CA) situated on both sides of the nostrils of the user P.

In the embodiment of Fig. 1-4, the mask body 10 comprises a front wall that is curved and/or flat, that is traversed by the front opening 11 that can have a circular shape or section and/or an inner diameter of about between 1 and 3 cm, for instance of between 15 and 25 mm. Preferably, the front wall of the mask body 10 has a thickness of between 2 and 6 mm.

The mask body 10 and the two arms 14 are made of plastic material and molded in one piece, for instance by injection molding or any other suitable molding process. Typically, the plastic or polymer material can be PP, PC, PBT or Nylon^{®}, or similar materials.

Furthermore, as shown in Figures 2 & 3, the mask body 10 further comprises several venting orifices 16, i.e. traversing holes, slots or similar, traversing the front wall and arranged around the front opening 11. They are configured for venting to the atmosphere at least a part of the CO₂-containing gaseous flow expired by the user P during his/her expiratory phases. The venting orifices 16 are preferably arranged in groups or arrays comprising several venting orifices 16. For instance, in 2 arrays that sandwich the front opening 11 of the mask body 10, i.e. arranged to the left and to right of the front opening 11. The venting orifices 16 are in fluid communication with the inner breathing chamber of the flexible cushion 20, via the tubular rear section 12. The venting orifices 16 can have a cylindrical, a tronconical or any other suitable shape.

Furthermore, the rear breathing opening has a (quasi-) rectangular or similar (e.g. trapezoidal shape). It is arranged at the center of the curved rear surface including the peripheral edge regions surrounding the rear breathing opening of the supple rear part 21 of the flexible nasal cushion 20. A bridge element, such as a little band or the like, dividing the rear breathing opening, namely forming a bridge between the upper and lower edges of the rear breathing opening, i.e. in the supple rear part 21. The bridge element can have an arch-shape, for instance a "U" or "V"-shape, that slightly projects into the inner breathing chamber of the cushion 20. When the nasal mask 1 is donned by the patient P, as shown in Fig. 1 & 4, the bridge element comes into contact with the outer region of the nose of the patient separating the nostrils, namely the nasal columella, so as to constitute a support or the like prohibiting a collapse, sagging or similar of the thin regions of the supple rear part 21 of the cushion 20. The bridge element 25 is preferably molded in one-piece with the rest of the flexible cushion 20.

The cushion 20 can exist in several sizes, such as small (S), medium (M) and/or large (L) sizes, so that it can fit various morphologies of patients.

As shown in Fig. 4, when donned by the patient P, the mask 1 further comprises a headgear 33 comprising flexible/elastic straps or similar structures.

The headgear 33 is attached to headgear-connecting structures 15, such as slots, hooks or the like, arranged at the free end of the two arms 24. The headgear 33 is used for correctly positioning, holding and/or securing the mask 1 on the head of the patient P. In other words, the headgear 33 can be adjusted to pull the mask 1 against the face/nose with sufficient force to achieve a gas tight seal between the mask 1 and the wearer's nasal regions as above explained.

In the frame of the present invention, the comfort of use for the patient of the mask 1 has been improved as below explained.

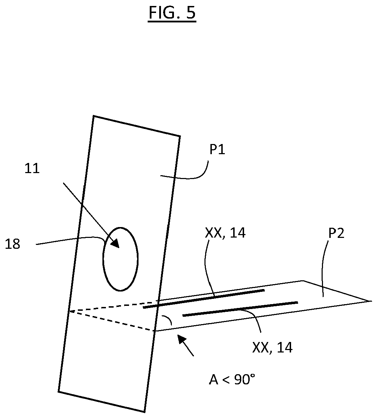

As shown in Fig. 3 & 5, the peripheral rim 18 of the front opening 11 of the mask body 10 can be considered as comprised in a first plane P1. Similarly, the two longitudinal axes (XX) of the elongated arms 14 can be considered as being comprised in a second plane P2.

Said first plane P1 and second plane P2 are inclined with respect to each other with an inclination angle A.

In a MCM-type mask 1 according to the prior art, especially the mask 1 taught by EP427910, the first plane P1 containing the rim 18 and the second plane (P0) containing the longitudinal axis (XX) of the two elongated arms 14 are perpendicular, i.e. their inclination angle A is of 90°. This is shown in Fig. 3, where P0 illustrates the second plane containing the two longitudinal axis (XX) of the elongated arms 14 of the mask of EP427910.

However, it has been noticed by the inventors of the present invention, in the frame of various empirical tests made with existing masks, that the discomfort that is often related to gas leaks, especially overnight, that can be due to a poor positioning of the mask of the patient's face and/or a poor tightness of the cushion or the like. In other words, the discomfort and/or leaks are closely related to the relative positioning of the mask 1 to the headgear 33.

Thus, when the first plane P1 containing the rim 18 is perpendicular to the second plane (P0) containing the longitudinal axis (XX) of the two elongated arms 14, the tension forces exerted by the flexible/elastic straps 34 of the headgear 33 are not sufficient, i.e. leading to leaks and discomfort. Further, the perpendicular inclination of the elongated arms with respect to the plane P1 containing the rim 18 leads to a non-perfect leverage effect leading to potential air leaks and to a discomfort for the patient as the cushion tends to collapse under its own weight until touching the upper lip of the patient and putting a pressure on it.

According to the present invention, to overcome this problem, the inventors have modified the two arms 14 so that their plane is not perpendicular to the plane of the rim 18.

As shown in Fig. 3 & 5, according to the present invention, the first plane P1, containing the rim 18 of the front opening 11 of the mask body 10, and the second plane P2, comprising the longitudinal axis (XX) of the two elongated arms 14, are inclined with respect to each other with an inclination angle (A) of less than 90°, i.e. there are not perpendicular.

Preferably, the inclination angle (A) is of at least 60° and/or of less than 88°, preferably of at least 65° and/or of less than 86°. More preferably, the inclination angle (A) is between about 70° and about 85°.

Choosing an inclination angle (A) of less than 90°, typically of between about 70° and about 85° as shown in Fig. 5, allows generating a leverage effect or similar due to the tension of the flexible/elastic traps 34 of the headgear 33 exerted on the two arms 24 and the mask body 10 that tends to pull up the cushion 20, in particular the regions at the bottom of the cushion 20 thereby improving the gas tightness and avoiding or limiting the gas leaks responsible for the discomfort of the patient P donning the mask 1.

In other words, thanks to the inclination angle (A) of less than 90°, it is possible to maintain the cushion 20 in a "raised" position that suppresses and/or limits the gas leaks, which is very important as prevention of air leaks is one of the most important features that a non-invasive ventilation (NIV) mask must fulfill.

The MCM-type mask 1 of the present invention can be used in a method for treatment of a respiratory disorder or condition affecting infant, toddler, child or adult patients, such as OSA or other respiratory troubles.

## Claims

1. Respiratory nasal mask (1) comprising:
- a mask body (10) comprising a front opening (11) with a peripheral rim (18), and two elongated arms (14), each elongated arm comprising a longitudinal axis (XX), and with the two elongated arms attached to opposite lateral sides (11a, 11b) of the mask body (10), and
- a hollow flexible cushion (20) comprising a supple rear part (21) comprising a rear breathing opening for allowing, in use, gas exchanges with nostrils of a user (P), said supple rear part (21) comprising peripheral edge regions surrounding the rear breathing opening that are configured to, in use, come into contact and provide a gas sealing with areas located immediately around the nostril edges of the user (P), the hollow flexible cushion (20) being detachably attached to the mask body (10),
**characterized in that** the peripheral rim (18) of the front opening (11) of the mask body (10) is comprised in a first plane (P1), and the longitudinal axis (XX) of the two elongated arms (14) are comprised in a second plane (P2), the first plane (P1) and the second plane (P2) being inclined with respect to each other with an inclination angle (A) of less than 90°.

2. Mask according to Claim 1, **characterized in that** the inclination angle (A) is of at least 60°, preferably of at least 65°.

3. Mask according to any one of the preceding Claims, **characterized in that** the inclination angle (A) is comprised between 70° and 85°.

4. Mask according to any one of the preceding Claims, **characterized in that** the two lateral arms (14) project on each lateral sides (11a, 11b) of the mask body (10) toward the cushion (20) so that the cushion (20) is sandwiched between said two lateral arms (14).

5. Mask according to any one of the preceding Claims, **characterized in that** two lateral arms (14) are made one-piece with the mask body (10).

6. Mask according to any one of Claims 1, 4 or 5, **characterized in that** the mask body (10) is made of polymer material.

7. Mask according to Claim 1, **characterized in that** the supple rear part (21) of the flexible cushion (20) comprises an upper lip area (ULA) located immediately under the nostrils of the patient (P), two curved alae (CA) on both sides of the nostrils of the patient (P) and a tip region (TR) of the nose of the patient (P), when the mask (1) is donned by the patient (P).

8. Mask according to any one of Claims 1 or 7, **characterized in that** the cushion (20) is made of silicone.

9. Mask according to Claim 1, **characterized in that** a tubular hollow connector (31) is secured to the front opening (11) of the mask body (10).

10. Mask according to Claim 1, **characterized in that** the peripheral rim (18) of the front opening (11) has a cylindrical section.

11. Mask according to Claim 1, **characterized in that** the two lateral arms (14) comprise headgear-connecting structures (15), preferably slots or hooks.

12. Mask according to Claim 11, **characterized in that** a headgear (33) is attached to the headgear-connecting structures (15).

13. Mask according to Claim 12, **characterized in that** the headgear (33) comprises elastic or flexible straps (34).

14. Mask according to Claim 9, **characterized in that** the tubular hollow connector (31) is rotatably secured to the front opening (11).

15. Installation for providing gas to a patient (P) comprising a medical ventilator, a respiratory nasal mask (1) according to any one of the preceding Claims, and a headgear (32) attached to the elongated arms (14) of the respiratory nasal mask (1), said medical ventilator being fluidly and mechanically connected to the respiratory nasal mask (1) by means of a flexible hose (30) comprising a tubular hollow connector (31).

## Amended claims

### Amended claims in accordance with Rule 137(2) EPC.

1. Respiratory nasal mask (1) comprising:
- a mask body (10) comprising a front opening (11) with a peripheral rim (18), and two elongated arms (14), each elongated arm comprising a longitudinal axis (XX), and with the two elongated arms attached to opposite lateral sides (11a, 11b) of the mask body (10), and
- a hollow flexible cushion (20) comprising a supple rear part (21) comprising a rear breathing opening for allowing, in use, gas exchanges with nostrils of a user (P), said supple rear part (21) comprising peripheral edge regions surrounding the rear breathing opening that are configured to, in use, come into contact and provide a gas sealing with areas located immediately around the nostril edges of the user (P), the hollow flexible cushion (20) being detachably attached to the mask body (10),
**characterized in that** the peripheral rim (18) of the front opening (11) of the mask body (10) is comprised in a first plane (P1), and the longitudinal axis (XX) of the two elongated arms (14) are comprised in a second plane (P2), the first plane (P1) and the second plane (P2) being inclined with respect to each other with an inclination angle (A) of between 70° and 85°.

2. Mask according to Claim 1, **characterized in that** the two lateral arms (14) project on each lateral sides (11a, 11b) of the mask body (10) toward the cushion (20) so that the cushion (20) is sandwiched between said two lateral arms (14).

3. Mask according to any one of the preceding Claims, **characterized in that** two lateral arms (14) are made one-piece with the mask body (10).

4. Mask according to any one of the preceding Claims, **characterized in that** the mask body (10) is made of polymer material.

5. Mask according to Claim 1, **characterized in that** the supple rear part (21) of the flexible cushion (20) comprises an upper lip area (ULA) located immediately under the nostrils of the patient (P), two curved alae (CA) on both sides of the nostrils of the patient (P) and a tip region (TR) of the nose of the patient (P), when the mask (1) is donned by the patient (P).

6. Mask according to any one of Claims 1 or 5, **characterized in that** the cushion (20) is made of silicone.

7. Mask according to Claim 1, **characterized in that** a tubular hollow connector (31) is secured to the front opening (11) of the mask body (10).

8. Mask according to Claim 1, **characterized in that** the peripheral rim (18) of the front opening (11) has a cylindrical section.

9. Mask according to Claim 1, **characterized in that** the two lateral arms (14) comprise headgear-connecting structures (15).

10. Mask according to Claim 9, **characterized in that** the headgear-connecting structures (15) are slots or hooks.

11. Mask according to Claim 9, **characterized in that** a headgear (33) is attached to the headgear-connecting structures (15).

12. Mask according to Claim 11, **characterized in that** the headgear (33) comprises elastic or flexible straps (34).

13. Mask according to Claim 7, **characterized in that** the tubular hollow connector (31) is rotatably secured to the front opening (11).

14. Installation for providing gas to a patient (P) comprising a medical ventilator, a respiratory nasal mask (1) according to any one of the preceding Claims, and a headgear (32) attached to the elongated arms (14) of the respiratory nasal mask (1), said medical ventilator being fluidly and mechanically connected to the respiratory nasal mask (1) by means of a flexible hose (30) comprising a tubular hollow connector (31).
